(19) Europäisches Patentamt
European Patent Office
Office européen des brevets

(11) Veröffentlichungsnummer: **0 336 267**
**A2**

# (12) EUROPÄISCHE PATENTANMELDUNG

(21) Anmeldenummer: **89105483.5**

(22) Anmeldetag: **28.03.89**

(51) Int. Cl.[4]: **C07C 91/26 , C07C 103/54 , C07C 103/70 , C11D 1/62 , C11D 1/65**

(30) Priorität: **02.04.88 DE 3811247**

(43) Veröffentlichungstag der Anmeldung: **11.10.89 Patentblatt 89/41**

(84) Benannte Vertragsstaaten: **ES**

(71) Anmelder: **Henkel Kommanditgesellschaft auf Aktien**
**Postfach 1100 Henkelstrasse 67**
**D-4000 Düsseldorf 1(DE)**

(72) Erfinder: **Rutzen, Horst, Dr.**
**Falkenweg 12**
**D-4018 Langenfeld(DE)**
Erfinder: **Baumann, Horst, Dr.**
**Vogelwarte 17**
**D-5653 Leichlingen(DE)**
Erfinder: **Ploog, Uwe, Dr.**
**Haydnweg 6**
**D-5657 Haan(DE)**
Erfinder: **Uphues, Günter**
**Robert-Koch-Strasse 45**
**D-4019 Monheim(DE)**

## (54) Quartäre Ammoniumverbindungen.

(57) Die Erfindung betrifft quartäre Ammoniumverbindungen mit einem gegebenenfalls langkettigen Carboxylat-, Sulfonat- oder Sulfat-Anion, ein Verfahren zu deren Herstellung und deren Verwendung zum Weichmachen von Textilien.Die quartären Ammoniumverbindungen enthalten lediglich ein oder zwei langkettige Alkylreste homöopolar an das Stickstoffatom gebunden. Man stellt die quartären Ammoniumverbindungen aus Salzen teriärer Amine unter anschließender Quarternierung mit Epoxyalkanen her.

EP 0 336 267 A2

## Quartäre Ammoniumverbindungen

Die Erfindung betrifft quartäre Ammoniumverbindungen, Verfahren zur deren Herstellung sowie deren Verwendung zum Weichmachen von Textilien.

Die zum Weichmachen im allgemeinen verwendeten quartären Ammoniumverbindungen enthalten üblicherweise zwei bis drei lange Alkyl- oder Hydroxyalkylreste, die mit dem zentralen Ammoniumstickstoffatom homöopolar verbunden sind. Dadurch ist jedoch ein relativ großer apparativer und kostenträchtiger Aufwand bei der Herstellung dieser Weichmacher notwendig.

Demgemäß besteht die Aufgabe der vorliegenden Erfindung darin, neue quartäre Ammoniumverbindungen zur Verfügung zu stellen, die gegenüber den bisher bekannten Ammoniumverbindungen des Standes der Technik einfacher herzustellen sind.

Überraschenderweise wurde gefunden, daß es nicht erforderlich ist, daß die gesamte Anzahl der langkettigen Alkyl- oder Hydroxyalkylreste mit dem zentralen Ammoniumstickstoffatom homöopolar verknüpft sein muß. Es reicht aus, nur einen oder zwei langkettige Alkylreste homöopolar an das Stickstoffatom zu binden. Der oder die restlichen langkettigen Alkylreste können durch die Ausbildung eines Salzes einer quartären Am moniumverbindung und einer geeigneten langkettigen Carbon-oder Sulfonsäure sowie eines Sulfates mit dem quartären Ammoniumstickstoffatom verbunden werden.

Gegenstand der vorliegenden Anmeldung sind somit quartäre Ammoniumverbindungen der allgemeinen Formel (I)

$$R^3 - \overset{R}{\underset{R^4}{N^+}} - R^1 \quad R^5{-}X^- \qquad (I)$$

wobei

R für einen geradkettigen und/oder verzweigtkettigen Alkylrest mit 1 bis 4 C-Atomen steht,

$R^1$ für einen geradkettigen und/oder verzweigtkettigen gesättigten und/oder einfach oder mehrfach ungesättigten Hydroxyalkylrest mit 2 bis 22 C-Atomen steht,

$R^3$ für einen Hydroxyalkylrest mit 2 bis 4 C-Atomen, einen Alkylrest mit 1 bis 22 C-Atomen, $R^2$-CO-Y-$(CH_2)_m$- oder HOOC-$R^6$-CO-Y-$(CH_2)_m$- steht, wobei Y für -NH- oder -O-, $R^2$ für einen geradkettigen und/oder verzweigtkettigen, gesättigten oder einfach und/oder mehrfach ungesättigten Kohlenwasserstoffrest mit 8 bis 18 C-Atomen, m für 2 oder 3 und $R^6$ für einen geradkettigen, gesättigten oder einfach oder mehrfach ungesättigten bivalenten Kohlenwasserstoffrest mit 6 bis 20 C-Atomen steht,

$R^4$ für einen geradkettigen und/oder verzweigtkettigen Alkylrest mit 1 bis 4 C-Atomen oder $R^2$-CO-Y-$(CH_2)_m$- steht, $R^5$ für einen geradkettigen und/oder verzweigtkettigen, gesättigten oder einfach oder mehrfach ungesättigten Kohlenwasserstoffrest mit 1 bis 22 C-Atomen steht und X für COO, $OSO_3$ oder für $SO_3$ steht.

Es wurde gefunden, daß die erfindungsgemäßen quartären Ammoniumverbindungen außerordentlich gute Weichmachereigenschaften für Textilien besitzen. Zudem ist nur ein relativ geringer apparativer und kostenmäßiger Aufwand bei der Herstellung dieser Weichmacherstoffe notwendig.

Gemäß einer bevorzugten Ausführungsform der vorliegenden Erfindung sind quartäre Ammoniumverbindungen dadurch gekennzeichnet, daß die Reste $R^3$ und $R^4$ gleich sind und jeweils für $R^2$-CO-Y-$(CH_2)_m$- stehen. Dies wird erfindungsgemäß dadurch erreicht, daß ein Polyamin mit reaktivem Wasserstoff, wie Methyldiisopropylentriamin, zunächst mit 2 Mol Alkansäure unter Bildung eines Diamids umgesetzt wird, das anschließend mit Ethylenoxid, Wasser und weiterer Fettsäure am tertiären Stickstoff in die quartäre Ammoniumverbindung überführt wird. In dem Falle, daß zur Amidierung die gleiche Alkansäure verwendet wird wie bei dem nachträglichen Schritt der Quaternierung, sind auch die Reste $R^5$, und $R^2$ gleich. Demgemäß besteht eine weitere Ausführungsform der vorliegenden Erfindung darin, daß die Reste $R^5$ und $R^2$ gleich sind.

Während im allgemeinen von langkettigen und/oder kurzkettigen N-Alkylaminen erfindungsgemäß ausgegangen wird, ist eine weitere Ausführungsform der vorliegenden Erfindung dadurch gekennzeichnet, daß man von N-Methylaminverbindungen ausgeht. Die daraus erhaltenen quartären Ammoniumverbindungen sind dadurch gekennzeichnet, daß R in der allgemeinen Formel (I) für einen Methylrest steht.

Der Begriff "Alkansäure" steht im Sinne der vorliegenden Erfindung für Alkancarbonsäuren, Alkylsulfate und Alkansulfonsäuren.

Die Überführung des tertiären Stickstoffatoms in das quartäre Stickstoffatom wird erfindungsgemäß mit Epoxyalkanen durchgeführt. Gemäß einer bevorzugten Ausführungsform wird die Quaternierung mit Hilfe von Ethylenoxid durchgeführt, wenn in dem Ausgangsamin noch keine langkettigen Reste vorhanden sind, so daß in der erfindungsgemäßen quartären Ammoniumverbindung ein N-2-Hydroxyethylrest vorliegt. Erfindungsgemäße quartäre Ammoniumverbindungen sind daher gemäß dieser Ausführungsform dadurch gekennzeichnet, daß $R^1$ in der allgemeinen Formel (I) für einen N-2-Hydroxyethylrest steht.

Die erfindungsgemäßen quartären Ammoniumverbindungen können gemäß den nachstehend beschriebenen Verfahrensvarianten erhalten werden.

Gegenstand der Erfindung ist daher auch das Verfahren zur Herstellung von quartären Ammoniumverbindungen, das dadurch gekennzeichnet ist, daß man

(a) bezogen auf 1 Mol einer Verbindung der allgemeinen Formel (II)

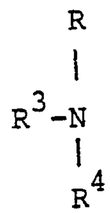

(II)

wobei R, $R^3$ und $R^4$ die oben genannten Bedeutungen haben, mit wenigstens 1 Mol Alkansäure der allgemeinen Formel (III)

$R^5$-X-H (III)

wobei $R^5$ und X die oben genannten Bedeutungen haben, mit einem Mol Epoxyalkan der allgemeinen Formel (IV)

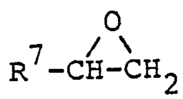

(IV)

wobei $R^7$ für einen geradkettigen und/oder verzweigtkettigen, langkettigen Alkylrest mit 6 bis 20 C-Atomen steht,

unter Quaternierung und Salzbildung umsetzt.

Soweit das Ausgangsamin der allgemeinen Formel (II) noch keine langkettigen Alkylreste homöopolar gebunden hat, wird das erfindungsgemäße Verfahren in einer bevorzugten Weise dadurch verwirklicht, daß ein Polyamin mit reaktivem Wasserstoff (beispielsweise Methyldi-n-propylentriamin) mit einer Fettsäure amidiert und anschließend mit einem Epoxyalkan (beispielsweise Ethylenoxid) in Gegenwart von Wasser und weiterer überschüssiger Fettsäure quaterniert wird. Das nachfolgende Reaktionsschema gibt den Reaktionsverlauf wieder.

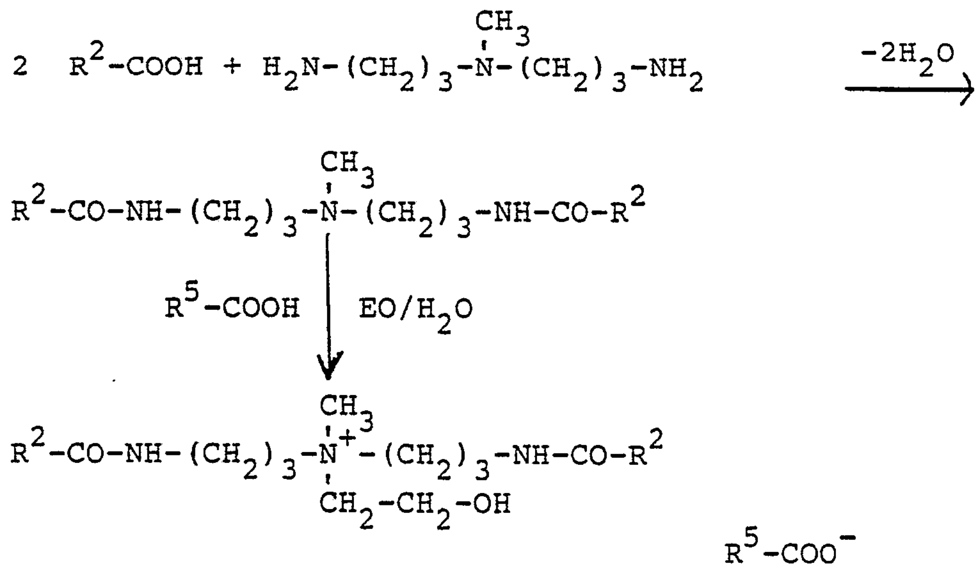

Die zur Amidierung eingesetzte Carbonsäure kann gleich der zur Quaternierung verwendeten Säure oder von ihr verschieden sein. In der Praxis ist es vorteilhaft, in beiden Verfahrensschritten die gleiche Säure einzusetzen, d.h. es kann $R^2 = R^5$ oder $R^2 \neq R^5$ sein.

Eine weitere erfindungsgemäße Verfahrensvariante zur Herstellung von quartären Ammoniumverbindungen ist dadurch gekennzeichnet, daß man bezogen auf 1 Mol einer Verbindung der allgemeinen Formel (II), im Fall, daß $R^3$ und $R^4$ für $-(CH_2)_m-NH_2$ steht, diese mit 3 Mol Alkansäure der allgemeinen Formel (III) und mit 1 Mol Epoxyalkan der allgemeinen Formel (IV) unter Quaternierung umsetzt.

Beispielsweise kann Methyldi-n-propylentriamin direkt mit 3 Mol Alkancarbonsäure umgesetzt werden, was sich wegen des 50 %igen Säureüberschusses sehr günstig auf die Reaktionsgeschwindigkeit und Vollständigkeit der Amidbildung auswirkt. Man erhält auf diese Weise das tertiäre Ammoniumsalz des Säureamids

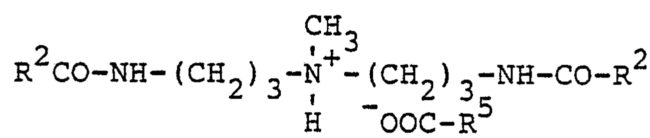

mit $R^2 = R^5$, das nach Zusatz von Wasser direkt mit Ethylenoxid oder einem anderen Epoxyalkan quaterniert werden kann. In der obigen Reaktionsfolge entstehen somit Verbindungen mit insgesamt drei langen Alkylresten, von denen eine ionogen und zwei homöopolar gebunden sind. Zur Ausbildung guter Weichspüleigenschaften genügen jedoch bereits zwei lange Alkylreste in dem Molekül, von denen einer ionogen verknüpft ist.

Eine weitere Verfahrensvariante der vorliegenden Erfindung besteht daher darin, daß man, bezogen auf 1 Mol einer Verbindung der allgemeinen Formel (V)

$$R^3-\underset{|}{\overset{R}{N}}-(CH_2)_m-Y-OC-R^2 \qquad (V)$$

(entsprechend Formel (II) mit R und $R^3$ = kurzkettiges Alkyl und $R^4 = -(CH_2)_m-Y-COR^2$), wobei m, R, $R^2$ und $R^3$ jeweils unabhängig voneinander die oben genannten Bedeutungen haben, diese mit 1 Mol eines Epoxyalkans der allgemeinen Formel (VI)

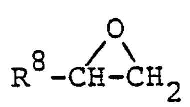
(VI)

in Gegenwart einer Alkansäure, $R^5$-X-H und Wasser umsetzt, wobei $R^8$ für einen geradkettigen und/oder verzweigtkettigen Alkylrest mit 1 bis 20 C-Atomen steht. Derartige Verbindungen sind somit beispielsweise

aus Dimethylpropylendiamin und Fettsäuren mit anschließender Quaternierung mit Ethylenoxid zugänglich. Das nachfolgende Reaktionsschema gibt den Verlauf des Herstellungsverfahrens gemäß dieser Verfahrensvariante wieder.

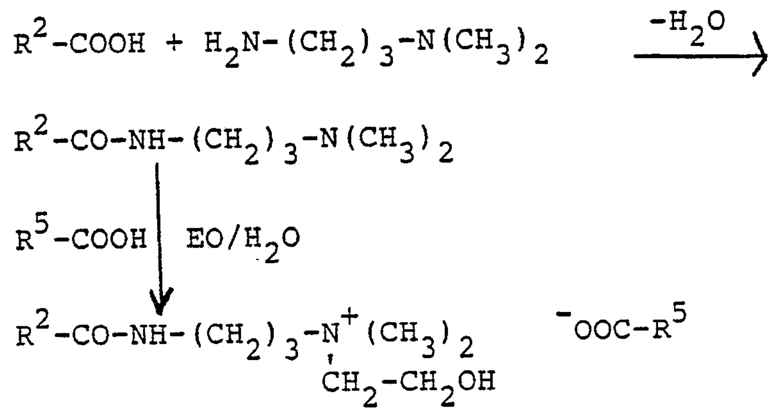


Auch hier ist es wiederum günstiger, für beide Reaktionsschritte die gleiche Carbonsäure $R^5 = R^2$ zu verwenden. Vorzugsweise wird die Amidierung im Molverhältnis (Säure : Amin) 2 : 1 durchgeführt, so daß man zunächst die Verbindungen der Formel (V) herstellt und diese mit 2 Mol Alkansäure der allgemeinen Formel (III) und Epoxyalkan der allgemeinen Formel (VI) umsetzt.

Die Überschneidung der allgmeinen Formeln (IV) und (VI) ist durchaus gewollt und dient der Unterscheidung von langkettigen Epoxyalkanen einerseits (Formel (IV)) und von sowohl kurzkettigen als auch langkettigen Epoxyalkanen andererseits (Formel (VI)).

Mit Hilfe der langkettigen Epoxyalkane der allgemeinen Formel (IV) können lange 2-Hydroxyalkylreste in quartäre Ammoniumverbindungen eingeführt werden. Der zweite lange Alkylrest ist demgemäß ionogen gebunden und stammt beispielsweise von einem Carbonsäureanion.

Das nachfolgende Reaktionsschema gibt den Reaktionsverlauf gemäß dieser Ausführungsform wieder.

Diese weitere Verfahrensvariante zur Herstellung der erfindungsgemäßen quartären Ammoniumverbindungen besteht darin, daß man, bezogen auf 1 Mol einer Verbindung der allgemeinen Formel (VII)

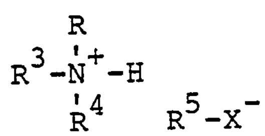
(VII)

wobei $R^5$ und X jeweils unabhängig voneinander die oben genannten Bedeutungen haben, $R^3$ für einen Hydroxyalkylrest mit 2 bis 4 C-Atomen und $R^4$ für einen geradkettigen und/oder verzweigtkettigen, gesättigten oder einfach oder mehrfach ungesättigten Kohlenwasserstoffrest mit 1 bis 4 C-Atomen steht, diese mit 1 Mol eines Epoxyalkans der allgemeinen Formel (IV) umsetzt.

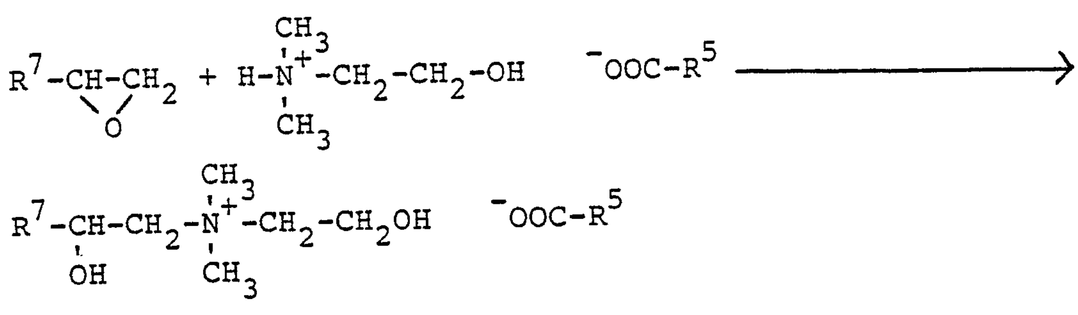

Weiterhin ist das Verfahren zur Herstellung von quartären Ammoniumverbindungen gemäß einer weiteren Ausführungsform der vorliegenden Erfindung dadurch gekennzeichnet, daß man, bezogen auf 1 Mol einer Verbindung der allgemeinen Formel (VIII),

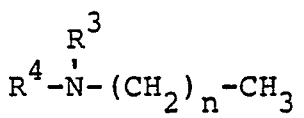 (VIII)

wobei
$R^3$ und $R^4$ jeweils unabhängig voneinander die oben genannte Bedeutung haben und
n für eine ganze Zahl im Bereich von 5 bis 23 steht,
diese mit 1 Mol Epoxyalkan der allgemeinen Formel (IV) und 1 Mol Alkansäure der allgemeinen Formel (III) umsetzt. Bei der Verwendung von langkettigen Alkansäuren, langkettigen Aminen und kurzkettigen Epoxyalkanen lassen sich somit zwei lange Alkylreste, von denen einer ionogen gebunden ist, in die quartäre Ammoniumverbindung einführen. Drei lange Alkylreste lassen sich durch die Verwendung von langkettigen Aminen und langkettigen Epoxyalkanen und langkettigen Alkansäuren realisieren.

Das nachfolgende Reaktionsschema beschreibt den Reaktionsverlauf gemäß dieser Ausführungsform.

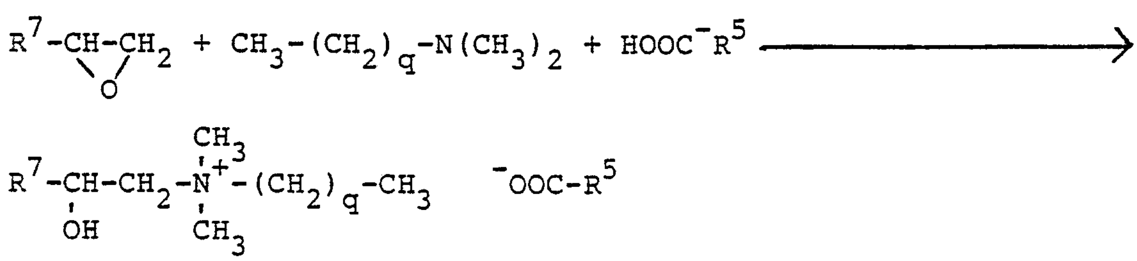

Ausgehend von Dicarbonsäuren der allgemeinen Formel (IX)
$HOOC-R^6-COOH$ (IX)
lassen sich bisquartäre Verbindungen der allgemeinen Formel (X)

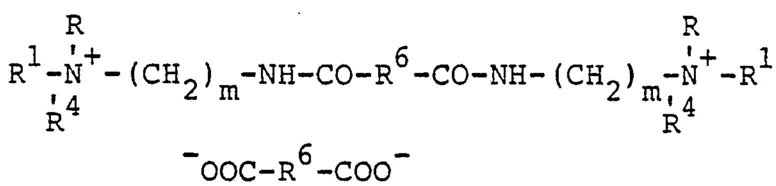 (X)

herstellen. Zu diesem Zweck wird die Dicarbonsäure mit einem Polyamin mit reaktivem Wasserstoff, beispielsweise Dimethylpropylendiamin im Stoffmengenverhältnis 1 : 2 umgesetzt und die entstandene Verbindung mit einem Epoxyalkan, beispielsweise Ethylenoxid, in Gegenwart eines weiteren Mols Dicarbonsäure, quaterniert. Demgemäß ist eine weitere Verfahrensvariante zur Herstellung von quartären Ammoniumverbindungen dadurch gekennzeichnet, daß man 2 Mol einer Verbindung der allgemeinen Formel (II), wobei $R^3$ für $-(CH_2)_m-NH_2$ steht, mit 1 Mol Dicarbonsäure der allgemeinen Formel (IX) umsetzt.

Eine weitere Variante des erfindungsgemäßen Verfahrens zur Herstellung von quartären Ammoniumverbindungen besteht darin, daß man 1 Mol einer Verbindung der allgemeinen Formel (II), wobei $R^3$ für $-(CH_2)_m-NH_2$ steht, mit 1 Mol Dicarbonsäure der allgemeinen Formel (IX) umsetzt. Das nachfolgende Reaktionsschema gibt den Reaktionsverlauf wieder.

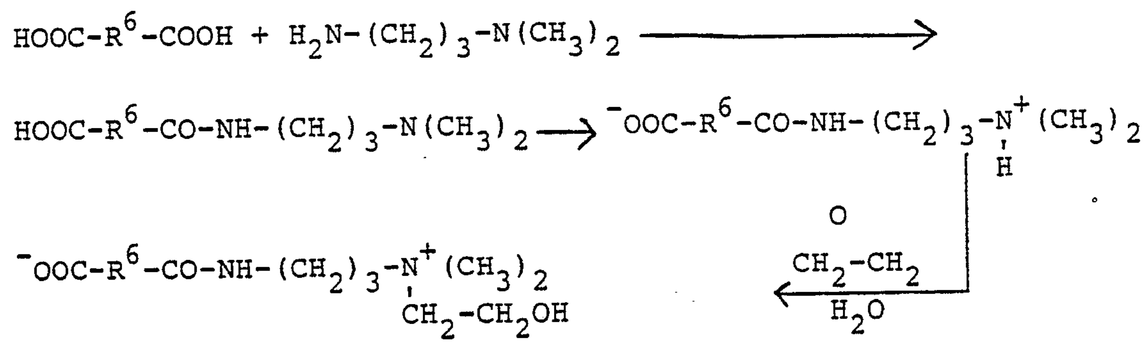

Anstelle von Ethylenoxid als Quaternierungsmittel in den erfindungsgemäßen Verfahren zur Herstellung von quartären Ammoniumverbindungen kann Propylenoxid, 1,2-Epoxydodecan oder 1,2-Epoxyhexadecan vorzugsweise als Epoxyalkan der allgemeinen Formeln (IV) und (VI) eingesetzt werden.

Sämtliche gemäß den oben genannten Verfahrensvarianten erhaltenen quartären Ammoniumverbindungen besitzen eine für die praktische Anwendung gute Weichspüler-Eigenschaft. Demgemäß ist die Verwendung der quartären Ammoniumverbindungen zum Weichmachen von Texilien ein weiterer Bestandteil der vorliegenden Erfindung.

## Beispiele

### Beispiel 1

#### a) Amidierung einschließlich Salzbildung

653,1 g (3 Mol) Kokos-12/18-Fettsäure (SZ (Säurezahl) 257,7 daraus ber. M (Molekulargewicht) 217,7), 145,3 g (1 Mol) Methyldipropylentriamin und 0,16 g (0,02 %, bezogen auf Gesamteinsatz) 50 %ige Phosphinsäure ($H_3PO_2$) wurden in einer Rührapparatur mit Wasserabscheider und Stickstoffabdeckung langsam auf 200 °C gebracht und 4 h bei dieser Temperatur gerührt. Während der Reaktion fielen 38 g Wasser an.

Das Reaktionsprodukt war ein gelbliches, festes Wachs. Kennzahlen: AZ (Aminzahl) 66,4; SZ 62,7 (berechnet: AZ = SZ = 73,6)

#### b) Quaternierung mit Ethylenoxid (EO)

202,8 g (0,24 Mol) Reaktionsprodukt gemäß Beispiel 1a) (M 844,9), 21.1 g (0,48 Mol) Ethylenoxid (100 % Überschuß) und 213,4 g Wasser wurden im Autoklaven unter Rühren auf 80 °C aufgeheizt und 2 h unter diesen Bedingungen (80 °C, 3 bar) gehalten. Weiße, weiche Paste. Kennzahlen: AZ 33,8 (berechnet: 31,5), SZ 0,52, Wasserbestimmung (K. Fischer) 44,4 %.

### Beispiel 2

#### a) Amidierung einschließlich Salzbildung

274,4 g (1 Mol) technische Ölsäure (SZ 204,4) wurden auf 150 °C aufgeheizt und 51,05 g (0,5 Mol) Dimethylpropylendiamin innerhalb 10 min zugetropft. Nach beendetem Zutropfen wurde die Temperatur innerhalb 2,5 h auf 200 °C gesteigert. Dabei sammelten sich 7,9 g Wasser im Wasserabscheider.

Das Reaktionsprodukt war flüssig und klar und besaß folgende Kennzahlen: SZ 82,9; AZ 87,2 (berechnet: AZ = SZ = 88,6)

b) Quaternierung mit EO

214,3 g (0,33 Mol) Reaktionsprodukt gemäß Beispiel 2a) (M 642,9, ber. aus AZ 87,2), 29,04 g (0,66 Mol) Ethylenoxid (100 % Überschuß) und 214,0 g Wasser wurden im Autoklaven bei 80 °C und 3 bar umgesetzt. Wie die stündliche Bestimmung der Säurezahl auswies, war die Reaktion nach 3 h beendet. Das Endprodukt bildete eine ca. 52 %ige gelbliche, weiche Paste. Kennzahlen: SZ 5,0; AZ 45,17 (berechnet: 42,2).

Beispiel 3

60,1 g (0,30 Mol) Laurinsäure, 26,7 g (0,30 Mol) Dimethylethanolamin, 56,4 g 1,2-Epoxydodecan (EpOZ (Epoxidzahl) 8,51, daraus ber. M 188,0) und 35,8 g destilliertes Wasser wurden zusammengegeben und in einer 500 ml Rührapparatur mit Stickstoffabdeckung auf 90 bis 95 °C erwärmt.

Nach 2,5 h war das Epoxid praktisch vollständig umgesetzt. Das Endprodukt fiel in Form einer ca. 80 %igen, weißen Paste an. Kennzahlen: Epoxidzahl (EpOZ) 0,06; SZ 5,1; AZ 96,9 (berechnet: 94,1).

Prüfung der Weichmachereigenschaften

Drei verschiedene bzw. verschieden vorbehandelte Textilien

a) gehärtetes Frottiergewebe
b) gehärtetes Moltongewebe
c) vorgewaschenes Frottiergewebe

wurden mit den zu prüfenden Substanzen, die in Form einer wäßrigen Dispersion (0,3 g/l) vorlagen, in einem Wackergerät imprägniert. Die Behandlungsdauer betrug 10 min. Anschließend erfolgte eine Trocknung der Textilien auf der Leine. Ein in Textilbewertungen erfahrener Personenkreis mit 6 Mitgliedern bestimmte die Griffnoten an jeweils zwei auf die gleiche Weise mit Wirkstoff vorbehandelten Gewebestükken.

Die Benotungsskala durchläuft 0 bis 6, wobei 0 sehr hart (Behandlung mit Wasser) und 6 sehr weich (Behandlung mit Ditalgalkyl-dimethylammoniumchlorid) bedeutet.

Tabelle 1

| Versuchs-Nr. | Ausgangsstoffe (Molverhältnis) | Griffnoten (0,3 g/l) | | | Durchschnittswert |
|---|---|---|---|---|---|
| | | gehärt. Frottier | gehärt Molton | vorgew. Frottier | |
| 1* | Laurinsäure-Methyldipropylentriamin-Ethylenoxid (3:1:1) | 3,5 | 3,9 | 3,8 | 3,7 |
| 2* | $C_{12}$-$C_{18}$Kokosölfettsäureschnitt Methyldipropylentriamin-Ethylenoxid (3:1:1) | 4,8 | 4,8 | 5,5 | 5,0 |
| 3* | Isopalmitinsäure aus $C_{16}$-Guerbetalkohol-Methyldipropylentriamin-Ethylenoxid (3:1:1) | 2,8 | 3,2 | 4,1 | 3,3 |
| 4* | $C_{16}$-$C_{18}$-Rindertalgfettsäure-Methyldipropylentriamin-Ethylenoxid (4:1:1) | 5,3 | 5,3 | 1,5 | 4,0 |
| 5* | Technische Ölsäure-Methyldipropylentriamin-Ethylenoxid (3:1:1) | 4,1 | 4,2 | 2,3 | 3,5 |
| 6** | Laurinsäure-Dimethylpropylendiamin-Ethylenoxid (2:1:1) | 4,1 | 3,3 | 3,8 | 3,7 |
| 7** | Isopalmitinsäure aus $C_{16}$-Guerbetalkohol-Dimethylpropylendiamin-Ethylenoxid (2:1:1) | 3,4 | 3,5 | 3,3 | 3,4 |
| 8** | Technische Ölsäure-Methylpropylendiamin-Ethylenoxid (2:1:1) | 4,9 | 3,8 | 4,4 | 4,3 |

* = Herstellung nach Beispiel 1

** = Herstellung nach Beispiel 2

Tabelle 2

| Versuchs-Nr. | Ausgangsstoffe (Molverhältnis) | Griffnoten (0,3 g/l) | | | Durchschnittswert |
|---|---|---|---|---|---|
| | | gehärt. Frottier | gehärt. Molton | vorgew. Frottier | |
| 9*** | 1,2-Epoxydodecan-Dimethylethanolamin-Laurinsäure (1:1:1) | 3,1 | 4,1 | 3,1 | 3,4 |
| 10*** | 1,2-Epoxydodecan-Dimethylethanolamin-Isotridecansäure (1:1:1) | 3,3 | 3,7 | 3,4 | 3,4 |
| 11*** | 1,2-Epoxydodecan-Dimethylethanolamin-Isopalmitinsäure aus $C_{16}$-Guerbetalkohol (1:1:1) | 3,1 | 3,1 | 3,1 | 3,1 |
| 12*** | 1,2-Epoxyhexadecan-Dimethylethanolamin-Essigsäure (1:1:1) | 3,4 | 3,7 | 3,7 | 3,6 |

*** Herstellung nach Beispiel 3

Beispiel 4

a)

76,7 g (0,25 Mol) $C_{12}$-Alkylbenzolsulfonsäure (ABSS), M306,84
22,3 g (0,25 Mol) Dimethylethanolamin (DMEA), M89,14
47,0 g (0,25 Mol) 1,2-Epoxydodecan (alpha-$C_{12}$), M188,0 (berechnet aus EpOZ) und
36,5 g destilliertes Wasser
wurden unter Rühren in einen Kolben gegeben, wobei sich das Reaktionsgemisch von selbst auf ca. 70 °C erwärmte. Nach vierstündiger Reaktion bei 95 °C waren die Komponenten praktisch vollständig umgesetzt. Auswaage: 167,2 g eines gelben, breiigen Produktes Kennzahlen: SZ 25,4; EpOZ 0,15; AZ 5,6.

Analog wurden die Produkte b), c) und d) hergestellt:

b)

76,7 g (0,25 Mol) ABSS
22,3 g (0,25 Mol) DMEA
66,1 g (0,25 Mol) alpha-$C_{16}$-Epoxid. ;264.5 (berechnet aus EpOZ 6,05)
41,3 g destilliertes Wasser
95 °C, 4,75 h
Auswaage: 195 g gelbe, steife Paste
Kennzahlen: SZ 19,7; EpOZ 0,18

c)

48,4 g (0,20 Mol) Hydropalmkern-Methylestersulfonsäure (HPK-MESS), M242,2 (berechnet aus SZ 231,6)
17,8 g (0,20 Mol) DMEA
37,6 g (0,20 Mol) alpha-$C_{12}$-Epoxid
26,0 g destilliertes Wasser
95 °C, 5 h
Auswaage: 117,1 g mittelbraune Paste
Kennzahlen: SZ 31,7, EpOZ 0,17, AZ 16,5

d)

48,4 g (0,20 Mol) HPK-MESS
17,8 g (0,20 Mol) DMEA
52,9 g (0,20 Mol) alpha-$C_{16}$-Epoxid
29,8 g destilliertes Wasser
95 °C, 4,5 h
Auswaage: 140,9 g gelbe Paste
Kennzahlen: SZ 27,7; EpOZ 0,27; AZ 16,6

Beispiel 5

Herstellung der Dicarbonsäurederivate

a) 145,0 g(0,376 Mol) Diacid (äquimolares Additionsprodukt aus Ölsäure und Acrylsäure der Fa. Westvaco), M385,5 (berechnet aus SZ 291,06) wurden auf 135 °C aufgeheizt und 38,4 g (0,376 Mol) Dimethylpropylendiamin (DMPDA), M102,2, unter Rühren innerhalb 30 min eingetropft. Innerhalb 2 h wurde die Temperatur auf 200 °C gesteigert, wobei sich 6,3 ml (berechnet 6,77 ml) Wasser abschieden. Auswaage: 171,5 g gelbes, zähflüssiges Produkt, SZ 102,8; AZ 108,9 (berechnet 119,5).
171,5 g (0,333 Mol) des erhaltenen Halbamides wurden mit 29,3 g (0,666 Mol) Ethylenoxid (EO), M44,05, in

200,0 g destilliertem Wasser bei 80 °C innerhalb 1 h im Rührautoklaven umgesetzt.
Auswaage: 363,6 g rotbraune Flüssigkeit, AZ 48,7.

b) 419,07 g (0,75 Mol) Dimerfettsäure (Dim), Pripol 1022 (dimerisierte Tallölfettsäure der Fa. Unichema), VZ 200,8 (daraus berechnet M 558,7) wurden auf 150 °C erhitzt und 76,51 g (0,75 Mol) DMPDA unter Rühren innerhalb 30 min zugetropft. Die Temperatur wurde in 40 min auf 200 °C gesteigert und 3 h eingehalten. Während dieser Zeit schieden sich 13,5 ml (berechnet 13,5 ml) Wasser ab.
Ausbeute ca. 460 g gelbes, klares, sehr zähflüssiges Produkt, AZ 83,2; SZ 78,7 (berechnet 87,2).
200,0 g (0,297 Mol) des erhaltenen Halbamids und 200,0 g destilliertes Wasser wurden mit 24,6 g (0,60 Mol) EO bei 80 °C und 3 bar in 2,25 h in einem Rührautoklaven behandelt.
Auswaage: 410 g hellfarbenes, zähflüssiges Produkt; SZ 2,0; AZ 39,8.

Beispiel 6

66,1 g (0,25 Mol) alpha-$C_{16}$-Epoxid (M264,5)
53,3 g (0,25 Mol) Dimethyldodecylamin ($C_{12}$-DMA), M213,35
50,1 g (0,25 Mol) Laurinsäure (M 200,32) und
42,4 g destilliertes Wasser
wurden 5 h bei 95 °C gerührt.
Auswaage: 202 g einer sehr viskosen, weißen Paste; SZ 5,9; EpOZ 0,17; AZ 66,0 (berechnet 66,3).

Die nachfolgende Tabelle 3 gibt in gleicher Weise wie Tabellen 1 und 2 die Griffnoten bei der Verwendung der erfindungsgemäßen quartären Ammoniumverbindungen wieder.

Tabelle 3

| Versuchs-Nr. | Ausgangsstoffe (Molverhältnis) | Griffnoten (0,3 g/l) | | | Durchschnittswert |
|---|---|---|---|---|---|
| | | gehärt. Frottier | gehärt. Molton | vorgew. Frottier | |
| 13**** | 1,2-Epoxydodecan-Dimethylethanolamin-$C_{12}$-Alkylbenzolsulfonsäure (1:1:1) | 3,2 | 3,0 | 2,8 | 3,0 |
| 14**** | 1,2-Epoxyhexadecan-Dimethylethanolamin-$C_{12}$-Alkylbenzolsulfonsäure (1:1:1) | 3,6 | 3,8 | 2,8 | 3,4 |
| 15**** | 1,2-Epoxydodecan-Dimethylethanolamin-Hydropalmkern-Methylestersulfonsäure (1:1:1) | 3,0 | 2,2 | 2,8 | 2,6 |
| 16**** | 1,2-Epoxyhexadecan-Dimethylethanolamin-Hydropalmkern-Methylestersulfonsäure (1:1:1) | 2,8 | 3,5 | 3,0 | 3,1 |
| 17***** | Diacid-Dimethylpropylendiamin-Ethylenoxid (1:1:1) | 3,3 | 2,4 | 2,7 | 2,8 |
| 18***** | Dimerfettsäure-Dimethylpropylenamin-Ethylenoxid (1:1:1) | 3,0 | 2,7 | 3,0 | 2,9 |
| 19****** | 1,2-Epoxyhexadecan-Dimethyldodecylamin-Laurinsäure (1:1:1) | 4,8 | 4,1 | 4,6 | 4,5 |

**** = Herstellung nach Beispiel 4
***** = Herstellung nach Beispiel 5
****** = Herstellung nach Beispiel 6

## Ansprüche

1. Quartäre Ammoniumverbindungen der allgemeinen Formel (I)

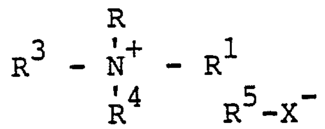

(I)

wobei

R für einen geradkettigen und/oder verzweigtkettigen Alkylrest mit 1 bis 4 C-Atomen steht,

$R^1$ für einen geradkettigen und/oder verzweigtkettigen gesättigten und/oder einfach oder mehrfach ungesättigten Hydroxyalkylrest mit 2 bis 22 C-Atomen steht,

$R^3$ für einen Hydroxyalkylrest mit 2 bis 4 C-Atomen, einen Alkylrest mit 1 bis 22 C-Atomen, $R^2$-CO-Y-$(CH_2)_m$- oder HOOC-$R^6$-CO-Y-$(CH_2)_m$- steht, wobei Y für -NH- oder -O-, $R^2$ für einen geradkettigen und/oder verzweigtkettigen, gesättigten oder einfach und/oder mehrfach ungesättigten Kohlenwasserstoffrest mit 8 bis 18 C-Atomen, m für 2 oder 3 und $R^6$ für einen geradkettigen, gesättigten oder einfach oder mehrfach ungesättigten bivalenten Kohlenwasserstoffrest mit 6 bis 20 C-Atomen steht,

$R^4$ für einen geradkettigen und/oder verzweigtkettigen Alkylrest mit 1 bis 4 C-Atomen oder $R^2$-CO-Y-$(CH_2)_m$-steht,

$R^5$ für einen geradkettigen und/oder verzweigtkettigen, gesättigten oder einfach oder mehrfach ungesättigten Kohlenwasserstoffrest mit 1 bis 22 C-Atomen steht und

X für COO, $OSO_3$ oder für $SO_3$ steht.

2. Quartäre Ammoniumverbindungen nach Anspruch 1, dadurch gekennzeichnet, daß $R^3$ und $R^4$ gleich sind und jeweils für $R^2$-CO-Y-$(CH_2)_m$ stehen.

3. Quartäre Ammoniumverbindungen nach Ansprüchen 1 oder 2, dadurch gekennzeichnet, daß $R^5$ und $R^2$ gleich sind.

4. Quartäre Ammoniumverbindungen nach Ansprüchen 1 bis 3, dadurch gekennzeichnet, daß R für einen Methylrest steht.

5. Quartäre Ammoniumverbindungen nach Ansprüchen 1 bis 4, dadurch gekennzeichnet, daß $R^1$ für einen N-2-Hydroxyethylrest steht.

6. Verfahren zur Herstellung von quartären Ammoniumverbindungen nach Ansprüchen 1 bis 5, dadurch gekennzeichnet, daß manbezogen auf 1 Mol einer Verbindung der allgemeinen Formel (II)

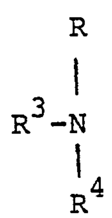

(II)

wobei R, $R^3$ und $R^4$ die oben genannte Bedeutung haben, mit wenigstens 1 Mol Alkansäure der allgemeinen Formel (III)

$R^5$-X-H (III)

wobei $R^5$ und X die oben genannte Bedeutung haben, und mit einem Mol Epoxyalkan der allgemeinen Formel (IV)

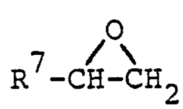

(IV)

wobei $R^7$ für einen geradkettigen und/oder verzweigtkettigen, langkettigen Alkylrest mit 6 bis 20 C-Atomen steht, unter Quaternierung und Salzbildung umsetzt.

7. Verfahren zur Herstellung von quartären Ammoniumverbindungen nach Anspruch 6, dadurch gekennzeichnet, daß man bezogen auf 1 Mol einer Verbindung der allgemeinen Formel (II), in dem Fall, daß $R^3$ für $-(CH_2)_m-NH_2$ steht, diese mit 3 Mol Alkansäure der allgemeinen Formel (III) und mit 1 Mol Epoxyalkan der allgemeinen Formel (IV) umsetzt.

8. Verfahren zur Herstellung von quartären Ammoniumverbindungen nach Ansprüchen 1 bis 5, dadurch gekennzeichnet, daß man, bezogen auf 1 Mol einer Verbindung der allgemeinen Formel (V)

$R^3-\overset{R}{\overset{|}{N}}-(CH_2)_m-Y-OC-R^2$ (V)

wobei m, R, $R^2$ und $R^3$ jeweils unabhängig voneinander die oben genannten Bedeutungen haben, diese mit 1 Mol eines Epoxyalkans der allgemeinen Formel (VI)

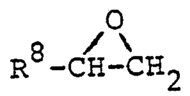 (VI)

wobei $R^8$ für einen geradkettigen und/oder verzweigtkettigen Alkylrest mit 1 bis 20 C-Atomen steht, umsetzt.

9. Verfahren zur Herstellung von quartären Ammoniumverbindungen nach Ansprüchen 1 bis 5, dadurch gekennzeichnet, daß man, bezogen auf 1 Mol einer Verbindung der allgemeinen Formel (VII)

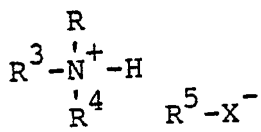 (VII)

wobei $R^5$ und X jeweils unabhängig voneinander die oben genannten Bedeutungen haben und $R^3$ für einen Hydroxyalkylrest mit 2 bis 4 C-Atomen und $R^4$ für einen geradkettigen und/oder verzweigtkettigen, gesättigten oder einfach oder mehrfach ungesättigten Kohlenwasserstoffrest mit 1 bis 4 C-Atomen steht, mit 1 Mol eines Epoxyalkans der allgemeinen Formel (IV) umsetzt.

10. Verfahren zur Herstellung von quartären Ammoniumverbindungen nach Ansprüchen 1 bis 5, dadurch gekennzeichnet, daß man, bezogen auf 1 Mol einer Verbindung der allgemeinen Formel (VIII)

$R^4-\overset{R}{\overset{|}{N}}-(CH_2)_n-CH_3$ (VIII)

wobei

$R^3$ und $R^4$ jeweils unabhängig voneinander die oben genannte Bedeutung haben und

n für eine ganze Zahl im Bereich von 5 bis 23 steht,

mit 1 Mol Epoxyalkan der allgmeinen Formel (IV) und 1 Mol Alkansäure der allgemeinen Formel (III) gleichzeitig umsetzt.

11. Verfahren zur Herstellung von quartären Ammoniumverbindungen nach Ansprüchen 1 bis 5, dadurch gekennzeichnet, daß man 1 Mol einer Verbindung der allgemeinen Formel (II), wobei $R^3$ für $-(CH_2)_m-NH_2$ steht, mit 1 Mol Dicarbonsäure der allgemeinen Formel (IX)

$HOOC-R^6-COOH$ (IX)

umsetzt.

12. Verfahren zur Herstellung von quartären Ammoniumverbindungen nach Anspruch 11, dadurch gekennzeichnet, daß man 2 Mol einer Verbindung der allgemeinen Formel (II) mit 1 Mol Dicarbonsäure der allgemeinen Formel (IX) umsetzt.

13. Verfahren zur Herstellung von quartären Ammoniumverbindungen nach Ansprüchen 6 bis 12, dadurch gekennzeichnet, daß man die Quaternierung mit Ethylenoxid, Propylenoxid, 1,2-Epoxydodecan oder 1,2-Epoxyhexadecan durchführt.

14. Verwendung von quartären Ammoniumverbindungen nach Ansprüchen 1 bis 5 zum Weichmachen von Textilien.